# EUROPEAN PATENT APPLICATION

(11) **EP 4 775 985 A1**
(43) Date of publication of application: **15.07.2026**
(21) Application number: 24888467.8
(22) Date of filing: 11.10.2024
(51) Int. Cl.: G01N 33/50, C07K 14/47, C07K 14/76, C07K 14/77, C07K 14/705, C12Q 1/68, G01N 33/48, G01N 33/53

(54) **STAIN TEST CONTROL SUBSTANCE, STAIN TEST SLIDE GLASS PREPARED USING SAME, STAIN TEST CONTROL SUBSTANCE PREPARATION KIT, AND METHOD FOR PRODUCING STAIN TEST CONTROL SUBSTANCE**

(30) Priority: 09.11.2023 JP 2023191310
(71) Applicant: Hitachi High-Tech Corporation, Minato-ku Tokyo 105-6409 (JP)
(72) Inventor: SASAKI Taira, Tokyo 100-8280 (JP); MATSUBARA Shigeki, Tokyo 105-6409 (JP)
(74) Representative: MERH-IP Matias Erny Reichl Hoffmann Patentanwälte PartG mbB
(86) International application number: PCT/JP2024/036516
(87) International publication number: WO 2025/100169

(57) **Abstract**

In order to provide a control substance to be used in a control test in a stain test, and a slide glass prepared using the control substance, and a control substance which is easy to prepare in a method for producing the control substance, and a slide glass prepared using the control substance, the following configuration was adopted.

A stain test control substance containing: a target molecule configured to specifically bind to a substance contained in a staining reagent; a charged molecule that is negatively charged; and a binding substance configured to bind the target molecule and the charged molecule.

## Description

### Technical Field

The present invention relates to a control substance to be used in a control test in a stain test, and a stain test slide glass prepared using the control substance, and more particularly, to a control substance that is less likely to peel off from a slide glass, a stain test slide glass prepared using the control substance, a stain test control substance preparation kit, and a method for producing a stain test control substance.

### Background Art

In a pathological examination for determining whether a subject is afflicted with a tumor such as cancer, a method is used in which a cell tissue collected from the subject is stained using a reagent that stains only a tumor, and when the cell tissue is stained, it is diagnosed that the subject is afflicted with the tumor. In this case, an error is not allowed in diagnosis by staining. Therefore, it is common to perform a positive and negative control test (control test) using a slide glass provided with a tissue (positive) reliably containing a tumor to be examined and a tissue (negative) not containing the tumor. A slide glass to be used in such a control test is commercially available.

PTL 1 discloses a control test slide glass, which is a slide glass for an immunostaining test to detect a test substance in a test sample using a labeled antibody, the slide glass including: a sample fixing portion that fixes the test sample; and a thin film portion including a thin film of a resin composition, on which a binding substance capable of forming a complex directly or indirectly with the labeled antibody and/or a nonbinding substance incapable of forming a complex with the labeled antibody are fixed.

### Citation List

### Patent Literature

PTL 1: JP2020-98097A

### Summary of Invention

### Technical Problem

In the technique described in PTL 1, the binding substance (a target molecule) is immobilized on the thin film of the resin composition. A coating material is described as an example of the resin composition, but it is assumed that the production is complicated, such as depositing a thin film of the resin composition onto a slide glass.

An object of the invention is to provide a control substance which is easy to prepare, a stain test slide glass prepared using the control substance, a stain test control substance preparation kit, and a method for producing a stain test control substance.

### Solution to Problem

To achieve the above object, the invention has the following configuration.

A stain test control substance containing: a target molecule configured to specifically bind to a substance contained in a staining reagent; a charged molecule that is negatively charged; and a binding substance configured to bind the target molecule and the charged molecule.

A stain test slide glass including: the stain test control substance adhering thereto.

A method for producing a stain test control substance, the method including: a step of aggregating a solution of a protein; a step of immersing an aggregate of the protein prepared in the previous step in a solution containing a target molecule to bind the target molecule; and a step of imparting a negative charge by chemically modifying the aggregate of the protein to which the target molecule is bound, which is prepared in the previous step.

A stain test control substance preparation kit including: a target molecule configured to specifically bind to a substance contained in a staining reagent; a charged molecule that is negatively charged; and a binding molecule configured to bind the target molecule and the charged molecule.

### Advantageous Effects of Invention

According to the invention, a control substance which is easy to prepare, a stain test slide glass prepared using the control substance, a stain test control substance preparation kit, and a method for producing a stain test control substance can be provided.

### Brief Description of Drawings

[FIG. 1] FIG. 1 is a diagram schematically illustrating a minimal configuration of a control substance of the invention.
[FIG. 2A] FIG. 2A is a diagram schematically illustrating a preparation scheme of the control substance of the invention (an example using an amino acid as a quenching agent).
[FIG. 2B] FIG. 2B is a diagram schematically illustrating a preparation scheme of the control substance of the invention (an example using a thiol group-containing amino acid of a mercaptopropionic acid as the quenching agent).
[FIG. 3] FIG. 3 is a diagram illustrating an example of a stain test slide glass according to the invention.
[FIG. 4] FIG. 4 illustrates an experimental result of Example 3.
[FIG. 5] FIG. 5 illustrates an experimental result of Comparative Example 2.
[FIG. 6A] FIG. 6A is a flowchart illustrating a method for producing a stain test control substance of the invention (part 1).
[FIG. 6B] FIG. 6B is a flowchart illustrating the method for producing a stain test control substance of the invention (part 2).

### Description of Embodiments

Hereinafter, an embodiment of the invention will be described with reference to the drawings. The following description shows a specific example of contents of the invention, the invention is not limited to the description, and various changes and modifications can be made by those skilled in the art within the scope of the technical idea disclosed in the description. In addition, in all the drawings for illustrating the invention, components having the same functions are denoted by the same reference signs, and repeated description thereof may be omitted.

A stain test slide glass provided with a control substance of the invention (hereinafter, also referred to as a "control substance") is preferably used for accuracy control (a check of whether a measurement is normally performed) of immunological analysis (a test of whether an antibody specific to a target is present in a specimen collected from a person) and a genetic test (a test of whether a gene specific to a target is present in a specimen) .

Particularly, when using an automatic staining device for analysis, unlike a manual method, a human adjustment of the analysis is not possible. Therefore, stable accuracy control is required for a control substance. When the substance is normally stained, it can be confirmed that a staining step normally proceeds, and when the substance is not normally stained, it can be confirmed that an abnormality occurs in the staining step.

As a target substance in the related art, a tissue collected from a human is generally used, but the control substance of the invention can be implemented based on design guidelines described below.

The control substance of the invention contains a target molecule that specifically binds to a substance contained in a staining reagent, a charged molecule that is negatively charged, and a binding substance that binds the target molecule and the charged molecule.

As the binding substance, any substance can be used as long as it can bind the target molecule and the charged molecule, and for example, a polymer or a copolymer containing a unit of acrylic acid, methacrylic acid, maleic acid, or maleic anhydride can be used.

As a more preferred binding substance, a protein may be cited as an example. This is because a specimen substance to be a comparison target of staining is biologically derived, making it preferable for the binding substance to also be biologically derived. Further, it is preferable for the protein to be aggregated. An aggregate of the protein is bound to a target molecule to be detected, and the aggregate of the protein is negatively charged.

The aggregate of the protein in the control substance of the invention is not particularly limited, and it is preferable that the aggregate of the protein has aggregability, exhibits low non-specific adsorption to an antibody reagent, and is readily available. Examples satisfying these conditions include serum albumin and ovalbumin.

The target molecule in the control substance of the invention can be appropriately changed depending on the application. For example, as a most preferred example, a protein molecule or a peptide molecule is used as the target molecule, and the control substance of the invention is used as a control substance for immunomorphological staining (IHC). In addition, by using a nucleic acid as the target molecule, the control substance of the invention can be used as a control substance for in situ hybridization (ISH).

Examples of the protein molecule used as the target molecule in the control substance of the invention include Ki-67, p53, CK, D2-40, CD20, CD3, ER, PGR, HER2, CD34, CD79, CD10, αSMA, S100, CD68, CK7, BCL2, CD56, p63, CD5, CK20, Synaptophysin, Vimentin, Chromogranin A, TTF-1, Desmin, CK-HMW, CD31, EMA, CD117, Cyclin D1, CD30, Myeloperoxidase, CD4, or CEA. Examples of the peptide molecule include a peptide molecule having a partial homologous sequence in the above protein molecule.

A method for binding the aggregate of the protein and the target molecule in the control substance of the invention is not particularly limited, and examples thereof include a method using a crosslinking agent.

From the viewpoint of imparting a negative charge to the protein, the crosslinking agent preferably binds to an amino group of the aggregate of the protein and to an amino group or a thiol group of the target molecule. As an example of satisfying such binding, a crosslinking method using an aldehyde-based or NHS maleimide-based crosslinking agent can be exemplified. The choice between the aldehyde-based crosslinking agent (for example, glutaraldehyde) or NHS maleimide can be made based on the application.

Since NHS maleimide is required to be controlled by pH at the time of crosslinking, the aldehyde-based crosslinking agent is generally preferred as the crosslinking agent. However, since the aldehyde-based crosslinking agent increases background fluorescence, the NHS maleimide-based crosslinking agent is preferred as the crosslinking agent when using a detection system using fluorescence such as FISH.

When a substance having only a negative charge is used as the binding substance instead of the aggregate of the protein, it is necessary to bind COOH of the binding substance to NH2 of the target molecule.

In this case, since it is difficult to use the aldehyde-based crosslinking agent and the NHS maleimide-based crosslinking agent, the following crosslinking agents are particularly preferably used. In this case, it is preferable to use the following quenching agents in combination.

### •Crosslinking Agent

A triazine-based crosslinking agent, a BOP-based crosslinking agent, and a carbodiimide-based crosslinking agent can be used. For example, DMT-MM, PyBOP, diisopropylcarbodiimide, dicyclohexylcarbodiimide, 1-ethyl-3-(3-dimethylaminopropyl)carbodiimid hydrochlorid, and 1-cyclohexyl-3-(2-morpholinoethyl)carbodiimid metho-p-toluolsulfonat

### •Quenching Agent

A substance containing an amine compound can be used. Examples thereof include a neutral amino acid molecule, an acidic amino acid molecule, and ethanolamine.

When the protein molecule or the peptide molecule is used as the target molecule, an amino group derived from lysine or a thiol group derived from cysteine can be used as a binding site. When the nucleic acid is used as the target molecule, an amino group or a thiol group may be previously introduced into the 3' or 5' end of the nucleic acid to serve as a binding site.

A method for imparting a negative charge to the aggregate of the protein is not particularly limited, and examples thereof include a method by chemical modification. Particularly, when the crosslinking agent is used in the binding of the aggregate of the protein and the target molecule, the negative charge can be imparted to the aggregate of the protein by reacting a quenching agent having a charge with an unreacted site of the crosslinking agent bound only to the aggregate of the protein.

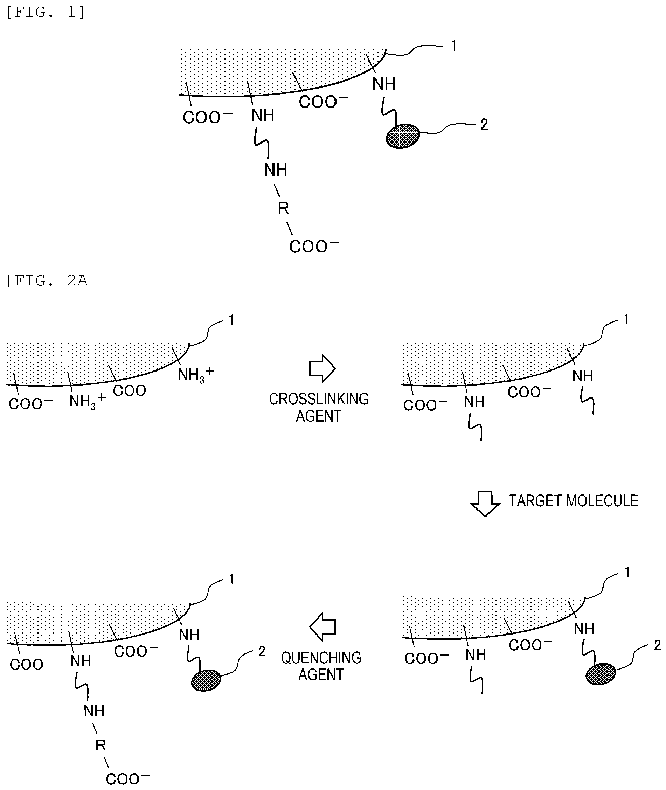

Representative examples are as follows. When the aldehyde-based crosslinking agent is used the crosslinking agent, the negative charge can be imparted by reacting the aldehyde-based crosslinking agent with an amino group of a neutral amino acid molecule or an acidic amino acid molecule as the quenching agent to introduce a carboxy group into the aggregate of the protein. In consideration of the fact that the aggregate of the protein is composed of amino acids, the above example of using amino acids as the quenching agent is particularly preferable in order not to change properties of the aggregate of the protein (FIG. 2A).

A peptide or an acidic peptide can also be used as the quenching agent, but chemical modification with a high molecular weight or application of an excessive amount of charge may inhibit the target reaction of the test.

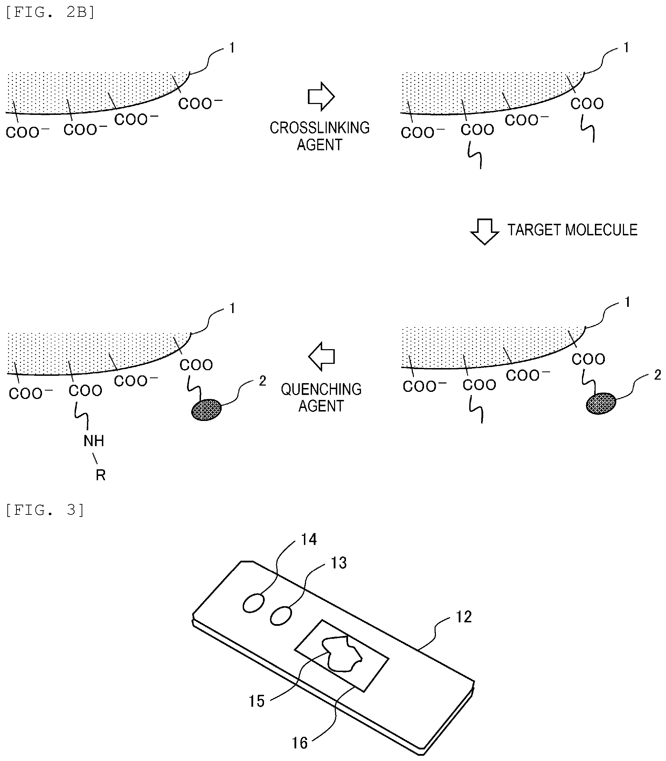

When the NHS maleimide-based crosslinking agent is used as the crosslinking agent, the negative charge can be imparted by reacting the NHS maleimide-based crosslinking agent with a thiol group of a mercaptopropionic acid as the quenching agent to introduce a carboxy group into the aggregate of the protein (FIG. 2B).

Examples of the crosslinking agent that can be used for binding to the target molecule of the invention include glyoxal, malondialdehyde, succinaldehyde, glutaraldehyde, and adipaldehyde, in the case of the aldehyde-based crosslinking agent. Glutaraldehyde, which is most widely used, is particularly preferred.

Examples of the crosslinking agent that can be used for binding to the target molecule of the invention include, in the case of the NHS maleimide-based crosslinking agent, 4-(N-maleimidomethyl)cyclohexanecarboxylic acid N-hydroxysuccinimide ester, 3-(maleimido)propionic acid N-hydroxysuccinimide ester, 6-(maleimido)hexanoic acid N-hydroxysuccinimide ester, maleimido-(PEG)ₙ-succinimide ester, and 3-maleimidobenzoic acid N-hydroxysuccinimide ester, or sulfo group-introduced derivatives thereof such as 4-(N-maleimidomethyl)cyclohexanecarboxylic acid N-hydroxysulfosuccinimide ester, 3-(maleimido)propionic acid N-hydroxysulfosuccinimide ester, 6-(maleimido)hexanoic acid N-hydroxysulfosuccinimide ester, maleimido-(PEG)ₙ-N-hydroxysulfosuccinimide ester, and 3-maleimidobenzoic acid N-hydroxysulfosuccinimide ester.

4-(N-Maleimidomethyl)cyclohexanecarboxylic acid N-hydroxysuccinimide ester, which is most widely used, is particularly preferred.

The crosslinking agent used for binding the target molecule of the invention is not limited to the aldehyde-based crosslinking agent and the NHS maleimide-based crosslinking agent described above. In addition to these, an anhydride-based crosslinking agent, an isothiocyanate-based crosslinking agent, an isocyanate-based crosslinking agent, a sulfonyl chloride-based crosslinking agent, an epoxide-based crosslinking agent, and a carbodiimide-based crosslinking agent can be used, and the negative charge can be imparted by treating the crosslinking agent with a corresponding quenching agent having a negative charge.

By using an acidic protein as the aggregate of the protein, the negatively charged aggregate of the protein can be obtained without depending on the chemical modification. In this case, either a charged or uncharged quenching agent can be used. The acidic protein is not particularly limited, and it is preferable that the acidic protein has aggregability, exhibits low non-specific adsorption to an antibody reagent, and is readily available. Examples satisfying these conditions include serum casein.

The strength of the negative charge of the aggregate of the protein can be evaluated by an isoelectric point. For example, a control substance is crushed and suspended, a zeta potential is measured while changing the pH, and a pH at which the zeta potential is 0 can be used as the isoelectric point of the aggregate of the protein. The lower the isoelectric point, the larger the negative charge of the aggregate of the protein.

An isoelectric point pI of the aggregate of the protein in the control substance of the present invention is preferably pI < 5, and more preferably pI < 4.5, considering that pI of bovine serum albumin or ovalbumin is around 5.

A method for producing a control substance of the invention is not particularly limited. Examples thereof include a method of aggregating a solution of a protein in a mold, binding a target molecule, imparting a negative charge by chemical modification to prepare a control substance, and slicing the control substance to a required thickness; a method of thinly applying a solution of a protein onto a fluorine-based plate-shaped mold, aggregating the solution of the protein, then binding a target molecule, imparting a negative charge by chemical modification to prepare a control substance, and cutting the control substance to a required area; and a method of spraying a solution of a protein onto a substrate such as a slide as in ink jet printing, aggregating the solution of the protein, then binding a target molecule, and imparting a negative charge by chemical modification to prepare a control substance.

A method for aggregating the solution of the protein is not particularly limited. Examples thereof include a method of aggregating by heating, a method of aggregating by adding an acid or an alkali, and a method of aggregating by adding an organic substance.

A shape of the control substance of the invention is not particularly limited. In consideration of uniformly applying heat and uniformly applying an immunostaining reagent, a cylindrical shape (a circular thin film) is preferred.

A thickness of the control substance of the invention is not particularly limited. A specimen used for immunostaining is generally about 5 µm, and a height of a liquid level of a staining reagent during immunostaining is generally about 100 µm, and therefore from the viewpoint of productivity and prevention of peeling from a slide, the thickness is preferably 0.5 µm or more and 500 µm or less, and more preferably 1 µm or more and 100 µm or less.

An area of the control substance of the invention is not particularly limited. From the viewpoint of productivity and visibility in inspection, the area is preferably 1 mm² or more and 1,000 mm² or less, and more preferably 5 mm² or more and 100 mm² or less.

The target molecule of the control substance of the invention can be histologically immobilized. The histological immobilization is a chemical treatment for protecting a specimen from deterioration due to autolysis or decay, and is often applied to immunohistochemical staining (IHC). One typical immobilization method is immobilization using formaldehyde. In the immobilization method, the target molecule is held for a long period of time in a state close to that in vivo by crosslinking the target molecule and a tissue component in the vicinity thereof with formaldehyde.

However, in the immobilization method, since a reaction site of an antigen is shielded, staining can be performed only by activation (deimmobilization) by a heat treatment or the like. By subjecting the control substance to histological immobilization similar to that of the specimen, it can be confirmed that both the steps of activation (deimmobilization) and staining are normal.

Hereinafter, the present embodiment will be specifically described with reference to Examples. The present embodiment is not limited to the following Examples.

### [Example 1]

FIG. 3 illustrates an example of a stain test slide glass according to the invention. A positive control substance 13 and a negative control substance 14 are provided on a slide glass 12. Further, a stain test sample 15 is provided on the slide glass 12 with paraffin 16.

In Example 1, a control substance of the invention was prepared.

Bovine serum albumin (10 wt%) was prepared and injected into a cylindrical mold having a diameter of 5 mm. The mold was heated at 90 °C to produce an aggregate of bovine serum albumin.

The aggregate was immersed in a 0.1 wt% glutaraldehyde solution and allowed to stand at room temperature for 3 hours.

The aggregate was immersed in a 100 µg/mL recombinant human vimentin solution (pH 7.4) and allowed to stand at room temperature overnight.

The aggregate was immersed in a 4% formaldehyde solution (pH 7.4) and allowed to stand at room temperature for 8 hours.

The aggregate was immersed in a 2% alanine aqueous solution and allowed to stand at room temperature overnight.

The aggregate was immersed in ethanol/water = 70 v/30 v% and allowed to stand overnight, then immersed in ethanol/water = 95 v/5 v% and allowed to stand for 1 hour, and then immersed in ethanol and allowed to stand for 3 hours.

The aggregate was immersed in xylene/ethanol = 70 v/30 v% and allowed to stand for 1 hour, then immersed in xylene/ethanol = 95 v/5 v% and allowed to stand for 1 hour, and then immersed in xylene and allowed to stand for 3 hours.

The aggregate was immersed in a xylene solution of 50 wt% paraffin and allowed to stand at 60 °C for 1 hour. The aggregate was immersed in paraffin, immersed at 60 °C for 3 hours, and then cooled at 4 °C to prepare a paraffin-embedded block of the aggregate.

The paraffin-embedded block of the aggregate was sliced with a microtome to prepare a control substance.

### [Example 2]

In Example 2, a control substance of the invention was prepared by a method different from that in Example 1.

Bovine serum albumin (10 wt%) was prepared and injected into a fluorine-based plate-shaped mold to a height of 200 µm.

The mold was heated at 90 °C to prepare a thin film-shaped aggregate of bovine serum albumin.

4-(N-maleimidomethyl)cyclohexanecarboxylic acid N-hydroxysuccinimide ester (0.1%, pH 8.5) was added dropwise to the mold and allowed to stand at room temperature for 3 hours, and the solution after the reaction was removed from the mold.

Mercaptoethylamine was added to a 1 mg/mL recombinant human vimentin solution (pH 6.0) and allowed to stand at 37 °C for 3 hours.

A protein component was purified by gel filtration to prepare a reduced product solution (pH 6.0) of 100 µg/mL of recombinant human vimentin.

The reduced product solution (pH 6.0) of 100 µg/mL of recombinant human vimentin was added dropwise to the mold and allowed to stand at room temperature overnight, and the solution after the reaction was removed from the mold.

The thin film-shaped aggregate was immersed in a 4% formaldehyde solution (pH 7.4) and allowed to stand at room temperature for 8 hours, and the solution after the reaction was removed from the mold.

The thin film-shaped aggregate was immersed in a 1% mercaptopropionic acid solution and allowed to stand at room temperature overnight, and the solution after the reaction was removed from the mold.

The thin film-shaped aggregate was cut into a size of 5 mm × 5 mm to prepare a control substance.

### [Example 3]

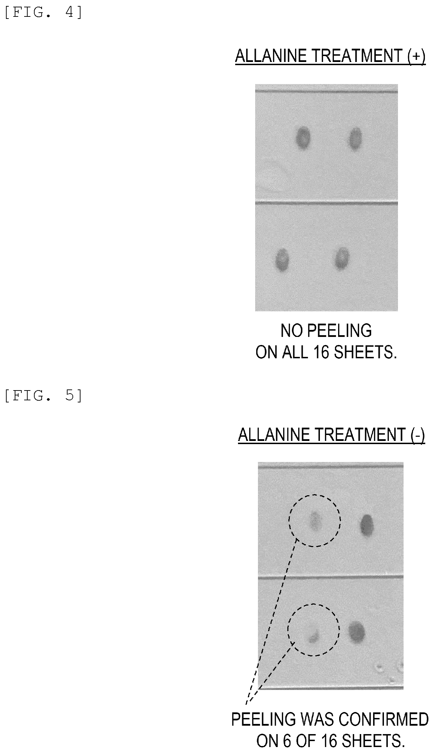

In Example 3, a quality control slide was prepared from the control substance prepared in Example 1 and immunostained with an automatic immunostaining device. It was confirmed that none of 16 control substances was peeled off from the slide. Favorable staining properties of all 16 control substances were confirmed (FIG. 4).

### <Comparative Example 1>

In Comparative Example 1, a comparative substance was prepared without controlling the charge by chemical modification.

Bovine serum albumin (10 wt%) was prepared and injected into a cylindrical mold having a diameter of 5 mm.

The mold was heated at 90 °C to produce an aggregate of bovine serum albumin.

The aggregate was immersed in a 0.1 wt% glutaraldehyde solution and allowed to stand at room temperature for 3 hours.

The aggregate was immersed in a 100 µg/mL recombinant human vimentin solution (pH 7.4) and allowed to stand at room temperature overnight.

The aggregate was immersed in a 4% formaldehyde solution (pH 7.4) and allowed to stand at room temperature for 8 hours.

The aggregate was immersed in ethanol/water = 70 v/30 v% and allowed to stand overnight, then immersed in ethanol/water = 95 v/5 v% and allowed to stand for 1 hour, and then immersed in ethanol and allowed to stand for 3 hours.

The aggregate was immersed in xylene/ethanol = 70 v/30 v% and allowed to stand for 1 hour, then immersed in xylene/ethanol = 95 v/5 v% and allowed to stand for 1 hour, and then immersed in xylene and allowed to stand for 3 hours.

The aggregate was immersed in a xylene solution of 50 wt% paraffin and allowed to stand at 60 °C for 1 hour. The aggregate was immersed in paraffin, immersed at 60 °C for 3 hours, and then cooled at 4 °C to prepare a paraffin-embedded block of the aggregate.

The paraffin-embedded block of the aggregate was sliced with a microtome to prepare a comparative substance.

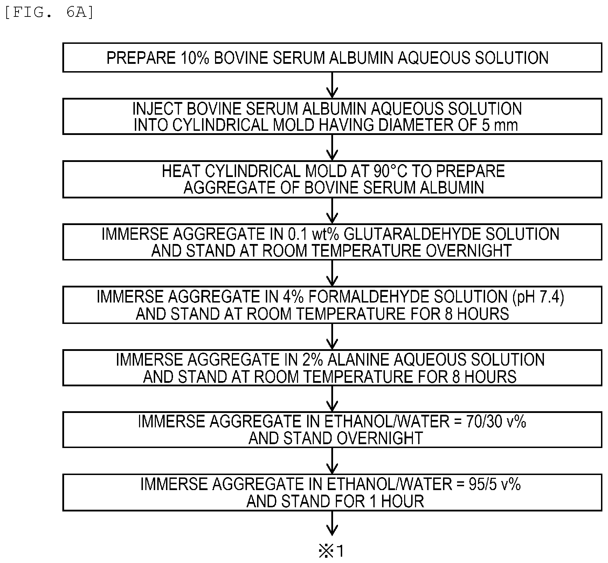

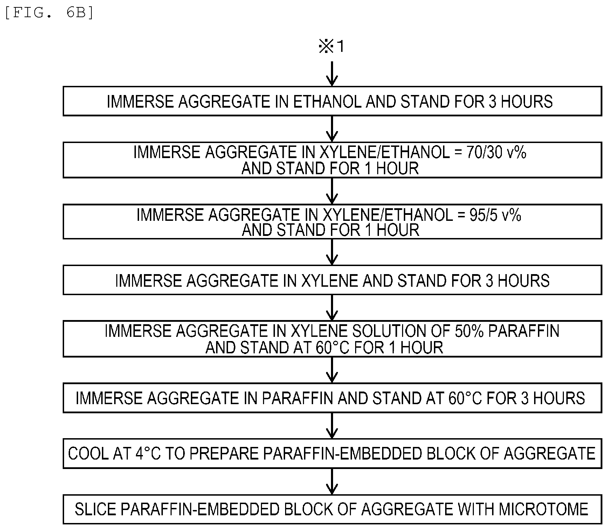

FIG. 6 illustrates a flowchart of preparation of the control substance according to the invention.

### <Comparative Example 2>

In Comparative Example 2, as a control experiment of Example 2, a comparative slide was prepared from the comparative substance prepared in Comparative Example 1, and immunostaining was performed with an automatic immunostaining device. It was confirmed that 6 of 16 comparative substances peeled off from the slide (FIG. 5).

### <Appendix>

The control (control) substance is a substance containing a target molecule to be detected in various tests, and is used for the purpose of test accuracy control. For example, in a test using staining, by evaluating a staining state of the control substance together with the specimen, whether the staining step in the test normally proceeds or whether an abnormality occurs can be confirmed. For example, since immunostaining in a pathological examination is used for diagnosis of cancer or the like, quality control is particularly important.

In order to stably supply a control substance at a lower cost, a technique for replacing a positive human tissue obtained by collecting the control substance from a human with an artifact is progressing.

In a clinical test, the spread of the automatic staining device is also progressing. Manual staining allows control over factors such as reagent drop placement and washing intensity, but such control is difficult with the automatic staining device, and the peeling of the control substance from the slide can become an issue.

When the control substance is peeled off, the validity of the staining step in the test cannot be determined. Therefore, it is expected that the demand for a control substance that is difficult to peel off from a slide increases.

According to the invention, a solution can be provided that makes it difficult for a control substance to peel off from a slide, thereby enabling relatively easy preparation of a control test slide glass.

### Reference Signs List

1 aggregate of protein
2 target molecule

## Claims

1. A stain test control substance comprising:
a target molecule configured to specifically bind to a substance contained in a staining reagent;
a charged molecule that is negatively charged; and
a binding substance configured to bind the target molecule and the charged molecule.

2. The stain test control substance according to claim 1, wherein
the binding substance is an aggregate of a protein.

3. The stain test control substance according to claim 2, wherein
the protein includes serum albumin or ovalbumin.

4. The stain test control substance according to claim 1, wherein
the target molecule is a protein molecule or a peptide molecule.

5. The stain test control substance according to claim 4, wherein
the protein molecule includes at least one of Ki-67, p53, CK, D2-40, CD20, CD3, ER, PGR, HER2, CD34, CD79, CD10, αSMA, S100, CD68, CK7, BCL2, CD56, p63, CD5, CK20, Synaptophysin, Vimentin, Chromogranin A, TTF-1, Desmin, CK-HMW, CD31, EMA, CD117, Cyclin D1, CD30, Myeloperoxidase, CD4, or CEA.

6. The stain test control substance according to claim 4, wherein
the peptide molecule is a peptide molecule having a partial homologous sequence in at least one molecule of Ki-67, p53, CK, D2-40, CD20, CD3, ER, PGR, HER2, CD34, CD79, CD10, αSMA, S100, CD68, CK7, BCL2, CD56, p63, CD5, CK20, Synaptophysin, Vimentin, Chromogranin A, TTF-1, Desmin, CK-HMW, CD31, EMA, CD117, Cyclin D1, CD30, Myeloperoxidase, CD4, or CEA.

7. The stain test control substance according to claim 2, wherein
the target molecule and the binding substance are bound by a crosslinking agent.

8. The stain test control substance according to claim 7, wherein
an amino group or a thiol group of the target molecule and an amino group of the aggregate of the protein as the binding substance are crosslinked by an aldehyde-based crosslinking agent or an NHS maleimide-based crosslinking agent.

9. The stain test control substance according to claim 1, wherein
the target molecule is a nucleic acid.

10. The stain test control substance according to claim 9, wherein
an amino group or a thiol group previously introduced into a 3' or 5' end of the nucleic acid is used as a binding site.

11. The stain test control substance according to claim 1, wherein
the charged molecule is imparted a negative charge by chemical modification.

12. The stain test control substance according to claim 11, wherein
the binding substance is a protein, and the negative charge is imparted by using a crosslinking agent and reacting a quenching agent having a charge with an unreacted site of the crosslinking agent bound only to the protein.

13. The stain test control substance according to claim 12, wherein
the crosslinking agent is an aldehyde-based crosslinking agent, the quenching agent is an amino group of a neutral amino acid molecule or an acidic amino acid molecule, and the negative charge is imparted by reacting the crosslinking agent with the quenching agent to introduce a carboxy group into an aggregate of the protein.

14. The stain test control substance according to claim 12, wherein
the crosslinking agent is an NHS maleimide-based crosslinking agent, the quenching agent is a thiol group of a mercapto fatty acid, and the negative charge is imparted by reacting the crosslinking agent with the quenching agent to introduce a carboxy group into an aggregate of the protein.

15. The stain test control substance according to claim 12, wherein
the crosslinking agent includes at least one of 4-(N-maleimidomethyl)cyclohexanecarboxylic acid N-hydroxysuccinimide ester, 3-(maleimido)propionic acid N-hydroxysuccinimide ester, 6-(maleimido)hexanoic acid N-hydroxysuccinimide ester, maleimido-(PEG)ₙ-succinimide ester, and 3-maleimidobenzoic acid N-hydroxysuccinimide ester, or sulfo group-introduced derivatives thereof such as 4-(N-maleimidomethyl)cyclohexanecarboxylic acid N-hydroxysulfosuccinimide ester, 3-(maleimido)propionic acid N-hydroxysulfosuccinimide ester, 6-(maleimido)hexanoic acid N-hydroxysulfosuccinimide ester, maleimido-(PEG)ₙ-N-hydroxysulfosuccinimide ester, and 3-maleimidobenzoic acid N-hydroxysulfosuccinimide ester.

16. The stain test control substance according to claim 1, wherein
the binding substance is an acidic protein.

17. The stain test control substance according to claim 2, wherein
an isoelectric point of the aggregate of the protein is pI < 5.

18. The stain test control substance according to claim 2, wherein
the aggregate of the protein is a thin film, and a surface area of the thin film is 1 mm² or more and 1,000 mm² or less.

19. The stain test control substance according to claim 2, wherein
the aggregate of the protein is a thin film, and a thickness of the thin film is 0.5 µm or more and 500 µm or less.

20. The stain test control substance according to claim 1, wherein
the target molecule is histologically immobilized.

21. A stain test slide glass comprising:
the stain test control substance according to claim 1 adhering thereto.

22. A stain test control substance preparation kit comprising:
a target molecule configured to specifically bind to a substance contained in a staining reagent;
a charged molecule that is negatively charged; and
a binding molecule configured to bind the target molecule and the charged molecule.

23. A method for producing a stain test control substance, the method comprising:
a step of aggregating a solution of a protein;
a step of immersing an aggregate of the protein prepared in the previous step in a solution containing a target molecule to bind the target molecule; and
a step of imparting a negative charge by chemically modifying the aggregate of the protein to which the target molecule is bound, which is prepared in the previous step.
